# EUROPEAN PATENT APPLICATION

(11) **EP 4 434 524 A1**
(43) Date of publication of application: **25.09.2024**
(21) Application number: 23382275.8
(22) Date of filing: 24.03.2023
(51) Int. Cl.: A61K 31/4709, A61P 9/00, A61P 9/14

(54) **TIE2 INHIBITORS FOR THE TREATMENT OF VENOUS MALFORMATION**

(71) Applicant: Fundación para la Investigación Biomédica del Hospital Universitario la Paz (FIBHULP), 28046 Madrid (ES)
(72) Inventor: LÓPEZ GUTIÉRREZ, Juan Carlos, 28046 Madrid (ES)
(74) Representative: Hoffmann Eitle

(57) **Abstract**

Targeted therapy has become the first therapeutic option in many patients with vascular anomalies, due to the low incidence of side effects and to avoiding morbimortality of more aggressive treatments. Some venous malformations have partially responded to *mTOR* or *PIK3CA* inhibitors although most venous malformations present a variant in *TEK.* We present the first patient with a severe venous malformation treated with a *TIE2* kinase inhibitor (rebastinib).

## Description

### TECHNICAL FIELD

The present inventio is directed to the treatment of venous malformations with TIE2 inhibitors. The present invention is thus directed to the medical field, in particular to methods of treatment.

### BACKGROUND

Historically, vascular anomalies were only treated when symptomatic or complicated and treatment was mainly surgical because medical therapies were limited and ineffective. From a predominantly surgical approach, we have shifted to a less aggressive focus in the management of these patients. Given the limitations to achieve complete cure of vascular anomalies, physicians are choosing a more conservative path to reduce the symptoms and avoid progression, decreasing morbimortality and long-term sequelae as well as improving the quality of life of these patients.

For the past decade, clinical and experimental research together with technological advances have enhanced our knowledge regarding the physiopathology, the histopathology and the genetics of vascular anomalies, expanding at the same time our arsenal of therapeutic options. In this sense, venous malformations (VMs) can be managed with different therapeutic options depending on the extension and symptoms including pressure garments, anticoagulation, surgical resections, diode laser or sclerotherapy. However, these treatments are not always suitable for extensive venous malformations refractory to other treatments

Rebastinib is a selective *TIE2* kinase inhibitor (endothelial-specific receptor, tyrosine kinase with immunoglobulin-like loops and epidermal growth factor homology domains-2 (Tie2)) that has been used in many clinical trials for solid tumors, usually adjuvant to other chemotherapy drugs, with good results in both efficacy and safety profile.

For the present invention, we herein present a case of a patient with a life-threatening facial venous malformation considered terminal and without other available therapeutic options. We report the first "off-label" successful use of a *TIE2* inhibitor (Rebastinib) in a vascular anomaly.

In this sense, and as indicated in the examples of the present specification, a male 28-year-old patient was referred with a cervicofacial venous malformation involving half-lower face, anterior neck and oral cavity. Despite multiple previous treatments, malformation progressed and patient presented at referral with severe facial deformity, daily episodes of pain and inflammation needing painkillers, and difficulty in speech and swallowing. Patient had a somatic variant in *TEK* (c.2740C>T; p.Leu914Phe) and rebastinib, that had demonstrated efficacy and safety in many clinical trials for solid tumors, was approved for compassionate use. Initial dose was 25 mg once daily orally, increasing until 50 mg once daily due to the good response in the malformation and the absence of side effects. After three months of treatment, venous malformation had decrease in size (10% volume in MRI) with smaller superficial phlebectasias and lightening in color. Patient also referred improvement in speech and swallowing and quality of life scores were better.

This case report provides the first direct evidence that *TIE2* inhibition is a safe and effective therapeutic strategy in patients with extensive venous malformations refractory to other treatments.

### DEFINITIONS

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present invention. All publications, patent applications, patents and other references mentioned herein are incorporated by reference in their entirety. The materials, methods, and examples disclosed herein are illustrative only and not intended to be limiting.

The terms and expressions used herein have the ordinary meaning as is accorded to such terms and expressions with respect to their corresponding respective areas of inquiry and study except where specific meanings have otherwise been set forth herein.

As used herein and in the appended claims, the singular forms "a," "and," and "the" include plural referents unless the context clearly dictates otherwise. Thus, for example, reference to "a method" includes a plurality of such methods and reference to "a dose" includes reference to one or more doses and equivalents thereof known to those skilled in the art, and so forth.

The term "about" or "approximately" means within an acceptable error range for the particular value as determined by one of ordinary skill in the art, which will depend in part on how the value is measured or determined, e.g., the limitations of the measurement system. For example, "about" can mean within 1 or more than 1 standard deviation, per the practice in the art. Alternatively, "about" can mean a range of up to 20%, or up to 10%, or up to 5%, or up to 1% of a given value. Alternatively, particularly with respect to biological systems or processes, the term can mean within an order of magnitude, for example, within 5-fold, and or for example, within 2-fold, of a value. Where particular values are described in the application and claims, unless otherwise stated the term "about" meaning within an acceptable error range for the particular value should be assumed.

The terms "individual," "host," "subject," and "patient" are used interchangeably to refer to an animal that is the object of treatment, observation and/or experiment. Generally, the term refers to a human patient, but the methods and compositions may be equally applicable to non-human subjects such as other mammals. In some embodiments, the terms refer to humans. In further embodiments, the terms may refer to children.

The term "therapeutically effective amount," as used herein, refers to any amount of a compound which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

The terms "treat," "treating" or "treatment," as used herein, refers to methods of alleviating, abating or ameliorating a disease or condition symptoms, preventing additional symptoms, ameliorating or preventing the underlying metabolic causes of symptoms, inhibiting the disease or condition, arresting the development of the disease or condition, relieving the disease or condition, causing regression of the disease or condition, relieving a condition caused by the disease or condition, or stopping the symptoms of the disease or condition either prophylactically and/or therapeutically.

The term "pharmaceutically acceptable," as used herein, refers a material, such as a carrier or diluent, which does not abrogate the biological activity or properties of the compounds described herein. Such materials are administered to an individual without causing undesirable biological effects or interacting in a deleterious manner with any of the components of the composition in which it is contained.

The term "pharmaceutically acceptable salt," as used herein, refers to a formulation of a compound that does not cause significant irritation to an organism to which it is administered and does not abrogate the biological activity and properties of the compounds described herein.

The terms "composition" or "pharmaceutical composition," as used herein, refers to a mixture of at least one compound or antibody as disclosed herein, with at least one and optionally more than one other pharmaceutically acceptable chemical components, such as carriers, stabilizers, diluents, dispersing agents, suspending agents, thickening agents, and/or excipients.

The term "carrier" applied to pharmaceutical compositions of the disclosure refers to a diluent, excipient, or vehicle with which an active compound (e.g., dextromethorphan) is administered. Such pharmaceutical carriers can be sterile liquids, such as water, saline solutions, aqueous dextrose solutions, aqueous glycerol solutions, and oils, including those of petroleum, animal, vegetable, or synthetic origin, such as peanut oil, soybean oil, mineral oil, sesame oil and the like.

Venous malformation (VM) arises from developmental defects of the vasculature and is characterized by massively enlarged and tortuous venous channels. Lesions grow commensurately causing deformity, chronic swelling, obstruction of vital structures, bleeding and pain. Most VMs are associated with the activating endothelial cell tyrosine kinase receptor TIE2 mutation L914F. Therapeutic options for VM are limited and ineffective while target therapy such as mTOR inhibitor rapamycin shows moderate efficacy.

### DESCRIPTION OF EMBODIMENTS

Patients with extensive and diffuse venous malformations require multimodal treatments throughout their life from more conservative options such as pressure garments, anticoagulation and analgesics to more aggressive alternatives like surgical resections, diode laser or sclerotherapy. Targeted therapy based on genetic results from affected tissue has become a new desirable approach to decrease morbimortality and sequelae from more invasive procedures. This case report provides the first direct evidence that *TIE2* inhibition is a safe and effective therapeutic strategy that can markedly improve the outcome and quality of life of patients with extensive venous malformations refractory to other treatments.

Tunica interna endothelial cell kinase (TIE2) is a cell-surface receptor tyrosine kinase that is expressed in endothelial cells and a subset of macrophages, called TIE2-expressing tumor-associated macrophages (TIE2+ macrophages or TEMs). The angiopoietin/TIE2 kinase signaling pathway is involved in tumor angiogenesis and lymphangiogenesis, as well as in cancer cell intravasation and metastasis, and is linked with poor outcome and recurrence in cancer patients. TIE2+ macrophages not only promote cancer growth, cancer cell survival, angiogenesis and motility but can also limit the efficacy of the tumor response to chemotherapy or radiotherapy.⁴

Rebastinib (DCC-2036) is a selective *TIE2* kinase inhibitor that has proven decrease of *TIE2-*expression macrophages, reduction of tumor vascular density, inhibition of tumor growth, invasion and metastases, as well as increase of survival in murine cancer models.⁴ A phase 1, first-in-human, single-agent study investigated rebastinib in relapsed or refractory chronic or acute myeloid leukemia to investigate the safety of rebastinib and establish the maximum tolerated dose (150 mg twice daily).⁵ Two phase 1b/2 studies have further assessed the safety, tolerability, antitumor activity, and pharmacokinetics of rebastinib in combination with paclitaxel or carboplatin in patients with advanced or metastatic solid tumors.⁶⁻⁷

The patient described above presented with a life-threatening cervicofacial venous malformations, refractory to available treatments at the moment, not eligible in any ongoing clinical studies. For the present invention, we used rebastinib as a compassionate use to treat the life-threatening cervicofacial venous malformations of the patient.

Although the follow-up period was short, we observed a remarkable response since the start of the treatment with decrease in size, reduction of superficial venous dilatations and lightening in color. There was also referred improvement in speech and swallowing and Karnofksy score was higher.

In the following three months from finalizing the treatment, we observed a low rate of side effects using this drug. Most common adverse effects described in previous clinical trials were muscular weakness (20-25%), myalgia (5%) and blurred vision (5%) and our patient did not present any of these nor did others not previously describe so far.

This case report provides the first direct evidence that inhibition is a safe and effective therapeutic strategy that can markedly improve the outcome and quality of life of patients with extensive venous malformations refractory to other treatments.

In one aspect, the present invention refers to a method of treating venous malformation (VM), comprising the step of administering a Tie-2 inhibitor to an individual in need thereof. In one aspect, the Tie-2 inhibitor is rebastinib (4-[4-[(5-tert-butyl-2-quinolin-6-ylpyrazol-3-yl)carbamoylamino]-3-fluorophenoxy]-N-methylpyridine-2-carboxamide) or a salt thereof.

In one aspect, the Tie-2 inhibitor, preferably rebastinib, may be administered in an amount sufficient to promote cell apoptosis in a venous malformation lesion and/or promote vascular channel regression.

In one aspect, the Tie-2 inhibitor, preferably rebastinib, may be administered at a dose of from about 10 mg once daily to 100 mg twice daily. Preferably the dose is about 25 mg once or twice daily to about 50 mg once or twice daily.

Preferably, the Tie-2 inhibitor, preferably rebastinib, may be administered according to the following regimen:
Dose level 1- Patient receives the Tie-2 inhibitor, preferably rebastinib, 25 mg once a day for about 1 month, preferably 28 days. If the patient tolerates treatment the patient can scale to dose level 2.
Dose level 2- Patient receives the Tie-2 inhibitor, preferably rebastinib, 25 mg twice per day for about 1 month, preferably 28 days. If the patient tolerates treatment the patient can scale to dose level 2.
Dose level 3- Patient receives the Tie-2 inhibitor, preferably rebastinib, 50 mg twice per day for about 1 month, preferably 28 days, or as long as clinical benefit is observed.

If the patient does not scale to dose level 2 or 3, they may continue at dose level 1 or 2 as long as clinical benefit is observed.

In one aspect, the Tie-2 inhibitor, preferably rebastinib, may be administered in an amount and for a duration sufficient to reduce a venous malformation lesion size and/or weight.

In one aspect, the Tie-2 inhibitor, preferably rebastinib, is administered in an amount and for a duration sufficient to reduce the number of ki-67 expressing proliferative cells as compared to pretreatment levels of ki-67 expressing proliferative cells.

In an embodiment of any of the previous aspects, said venous malformation may be inherited cutaneomucosal venous malformation (VMCM) or blue rubber bleb nevus syndrome (BRBNS). Preferably, said venous malformation is cervicofacial venous malformation preferably involving half-lower face, anterior neck and/or the oral cavity.

In one aspect, a method of promoting VM lesion regression and/or reducing VM lesion expansion in an individual in need thereof is disclosed. In this aspect, the method may comprise the step of administering the Tie-2 inhibitor, preferably rebastinib. In this aspect, the Tie-2 inhibitor, preferably rebastinib, is administered orally, intravenously, intralesionally, or a combination thereof. In one aspect, the Tie-2 inhibitor, preferably rebastinib, may be administered orally or by direct lesional injection.

### Dosage

In one aspect, the agent disclosed herein, the Tie-2 inhibitor, preferably rebastinib, may be present in a composition, preferably a pharmaceutical composition, in an amount of from about 0.5% to about 95%, or from about 1% to about 90%, or from about 2% to about 85%, or from about 3% to about 80%, or from about 4%, about 75%, or from about 5% to about 70%, or from about 6%, about 65%, or from about 7% to about 60%, or from about 8% to about 55%, or from about 9% to about 50%, or from about 10% to about 40%, by weight of the composition.

The composition may be administered in oral dosage forms such as tablets, capsules (each of which includes sustained release or timed release formulations), pills, powders, granules, elixirs, tinctures, suspensions, syrups, and emulsions. It may also be administered in intravenous (bolus or infusion), intraperitoneal, subcutaneous, intralesional, or intramuscular forms all utilizing dosage forms well known to those of ordinary skill in the pharmaceutical arts. The composition may be administered by intranasal route via topical use of suitable intranasal vehicles, or via a transdermal route, for example using conventional transdermal skin patches. A dosage protocol for administration using a transdermal delivery system may be continuous rather than intermittent throughout the dosage regimen.

A dosage regimen will vary depending upon known factors such as the pharmacodynamic characteristics of the agents and their mode and route of administration; the species, age, sex, health, medical condition, and weight of the patient, the nature and extent of the symptoms, the kind of concurrent treatment, the frequency of treatment, the route of administration, the renal and hepatic function of the patient, and the desired effect. The effective amount of a drug required to prevent, counter, or arrest progression of a symptom or effect of a muscle contracture can be readily determined by an ordinarily skilled physician

The pharmaceutical composition may include suitable dosage forms for oral, parenteral (including subcutaneous, intramuscular, intradermal and intravenous), transdermal, sublingual, bronchial or nasal administration. The pharmaceutical compositions are prepared by conventional techniques appropriate to the desired preparation containing appropriate amounts of the active ingredient, that is, the Tie-2 inhibitor, preferably rebastinib, or a pharmaceutically acceptable salt thereof according to the invention.

The dosage of the Tie-2 inhibitor, preferably rebastinib, disclosed herein used to achieve a therapeutic effect will depend not only on such factors as the age, weight and sex of the patient and mode of administration, but also on the degree of inhibition desired and the potency of an agent disclosed herein for the particular disorder or disease concerned. It is also contemplated that the treatment and dosage of an agent disclosed herein may be administered in unit dosage form and that the unit dosage form would be adjusted accordingly by one skilled in the art to reflect the relative level of activity. The decision as to the particular dosage to be employed (and the number of times to be administered per day) is within the discretion of the physician and may be varied by titration of the dosage to the particular circumstances of this invention to produce the desired therapeutic effect.

The following examples merely illustrate the present invention, but they do not limit the same.

### EXAMPLE

### Case Report

### Case Report

A male patient, aged 28 years old, is referred to our center due to progression of a massive cervicofacial venous malformation despite multiple treatments. At birth, after a controlled and uneventful pregnancy, patient was diagnosed with a cervicofacial venous malformation involving half-lower face, anterior neck and oral cavity. During childhood and early adulthood patient underwent over 250 procedures comprising sclerotherapy and laser at his hospital of origin. Despite all these treatments, the venous malformation progressed and patient presented at referral with severe facial deformity, daily episodes of pain and inflammation needing painkillers, and difficulty in speech and swallowing severe obstructive sleep apnea syndrome.

At our center we decide to start sirolimus treatment but patient referred severe mucositis and acne that was not controlled with dose reduction and adjuvant treatment, eventually leading to withdrawal of sirolimus after two months of treatment. Then we tried to improve patient's symptoms and size of the malformation with diode laser, but major postoperative edema forced us to perform a tracheostomy, that could be removed a few months later. Karnofsky performance status score, a validated scale to assess general health status in patients with serious illness, was 50%.³

Due to these life-threatening complications, the previous therapeutic failure and the absence of any other effective treatment, we decided to explore the possibility of targeted therapy since patient presented a somatic variant in *TEK* (c.2740C>T; p.Leu914Phe). Rebastinib, a *TIE2* kinase inhibitor, had demonstrated efficacy and safety in many clinical trials for solid tumors so we requested a compassionate use treatment for our patient after signature of informed consent. Initially daily oral rebastinib was started at 25 mg once daily (qd). In the first month we observed a rapid improvement in the general status of the patient without clear reduction in malformation size, but improvement in daily pain and inflammation. Dose was increased to 25 mg twice daily (BID) with slight reduction in malformation size as well as decrease in venous dilatations and lightening of superficial malformation. Patient also referred improvement in speech and swallowing. Dose was again increased to 50 mg twice daily (BID), the lowest dose used in clinical trials, and improvement continued. Patient's neck circumference was reduced by 15% and MRI confirmed venous malformation shrinkage (10% volume decrease) at 3 months of treatment. The patient's Karnofsky score increased to 80% and he did not present any adverse effect. There was no evidence of organ toxicity as assessed by heart, liver, kidney function and blood testing. The patient is currently still under rebastinib treatment.

### References

1. Triana P, López Gutiérrez JC. Drug treatment of vascular anomalies. Cir Pediatr. 2020; 33(1): 3-10
2. Limaye N, Kangas J, Mendola A, Godfraind C, Schlögel MJ, Helaers R, et al. Somatic Activating PIK3CA Mutations Cause Venous Malformation. Am J Hum Genet. 2015; 97(6): 914-921
3. Péus D, Newcomb N, Hofer S. Appraisal of the Karnofsky Performance Status and proposal of a simple algorithmic system for its evaluation. BMC Med Inform Decis Mak. 2013; 13: 72
4. Harney AS, Karagiannis GS, Pignatelli J, Smith BD, Kadioglu E, Wise SC, et al. The Selective Tie2 Inhibitor Rebastinib Blocks Recruitment and Function of Tie2Hi Macrophages in Breast Cancer and Pancreatic Neuroendocrine Tumors. Mol Cancer Ther. 2017; 16(11): 2486-2501
5. Cortes J, Talpaz M, Smith HP, Snyder DS, Khoury J, Bhalla KN, et al. Phase 1 dose-finding study of rebastinib (DCC-2036) in patients with relapsed chronic myeloid leukemia and acute myeloid leukemia. Haematologica. 2017; 102(3): 519-528
6. Abstract B055: Phase 1b/2 study of rebastinib (DCC-2036) in combination with paclitaxel: Preliminary safety, efficacy, pharmacokinetics and pharmacodynamics in patients with advanced or metastatic solid tumors. Mol Cancer Ther. 2019; 18(12suppl): B055. Identifier: NCT03601897. https://clinicaltrials.gov/ct2/show/NCT03601897
7. Abstract 558P: A phase I study of rebastinib and carboplatin in patients with metastatic solid tumours. Ann Oncol. 2020; 31(4suppl): S481-S482 Identifier: NCT03717415. https://clinicaltrials.gov/ct2/show/NCT03717415

## Claims

1. A composition comprising a Tie-2 inhibitor for use in a method of treating venous malformation (VM).

2. The composition for use according to claim 1, wherein the Tie-2 inhibitor is rebastinib (4-[4-[(5-tert-butyl-2-quinolin-6-ylpyrazol-3-yl)carbamoylamino]-3-fluorophenoxy]-N-methylpyridine-2-carboxamide) or a salt thereof.

3. The composition for use according to claim 1, wherein the Tie-2 inhibitor is rebastinib and is administered at a dose of from about 10 mg to 100 mg once or twice daily.

4. The composition for use according to claim 1, wherein the Tie-2 inhibitor is rebastinib and is administered at a dose of from about 25 mg to 50 mg once or twice daily.

5. The composition for use according to claim 1, wherein the Tie-2 inhibitor is rebastinib and is administered at a dose of from about 25 mg to 50 mg once or twice daily during a period of at least one month.

6. The composition for use according to claim 1, wherein the Tie-2 inhibitor is rebastinib and is administered at a dose of from about 25 mg to 50 mg once or twice daily during a period of at one to 6 months, preferably about three months.

7. The composition for use according to any of claims 1 to 6, wherein said venous malformation is inherited cutaneomucosal venous malformation (VMCM) or blue rubber bleb nevus syndrome (BRBNS).

8. The composition for use according to any of claims 1 to 6, wherein said venous malformation, wherein said venous malformation is cervicofacial venous malformation.
